# EUROPEAN PATENT APPLICATION

(11) **EP 0 940 124 A1**
(43) Date of publication of application: **08.09.1999**
(21) Application number: 98600006.5
(22) Date of filing: 12.03.1998
(51) Int. Cl.: A61B 17/64

(54) **External fixation device with changeable angle for trochanteric fractures**

(30) Priority: 06.03.1998 GR 98010093
(71) Applicant: Christodoulou, Nikolaos, 36100 Karpenissi (GR)
(72) Inventor: Christodoulou, Nikolaos, 36100 Karpenissi (GR)

(57) **Abstract**

The external fixation device is used in surgical orthopaedics and traumatology for stabilization (osteosynthesis) of fractures, osteotomies, arthrodesis e.t.c.

Specially, in trochanteric fractures, until now, there is not described any special external nail which can permit compression or sliding at the area of the trochanteric fracture to increase stabilization and consolidation.

In internal osteosynthesis, nails or plates are placed internally with serious hemorrhagic open intervention without the nail or the plate being left outside of the skin.

The disclosure gives the possibility, without serious hemorrhagic open intervention, to place a special nail which is left outside the skin to be connected with an external fixation device in such a way as to cause compression or sliding on the trochanteric fracture in a direction parallel with the nail, and also it is possible to correct, if it is needed post-operatively, varus or valgus deformity.

It is also possible easily post-operatively to insert or extract the nail as it has a screwed end for the stabilization of the head and the neck of the femoral bone and this is impossible with internal nails without open hemorrhagic intervention.

The external fixation device with changeable angle for trochanteric fractures, as disclosed, is characterized mainly by that it includes a long external nail threaded at one end which can cause compression or sliding on a trochanteric fracture .

## Description

The patent is related to an external fixation device with changeable angle for trochanteric fractures that is with the possibility of changing the osteosynthesis angle in varus or valgus position and it is characterized by that it brings a special external screwed nail to ask compression or sliding at the trochanteric fracture area.

Until now, different kinds of metal devices are used for osteosynthesis of the trochanteric fractures as angular plates, internal sliding nails, etc. which are applied internally of the skin , totally , with serious open hemorrhagic intervention and generally with serious overload for the usually elderly patients which sustain these fractures . Except this disadvantage, in some cases, these materials penetrate the hip joint from the reason of osteoporosis and a second intervention is needed to correct this complication . Also a second serious open intervention is needed to correct possible varus deformity of the fracture .

The external fixation systems which have used for osteosynthesis of the trochanteric fractures have the disadvantage that they can not ask compression or sliding at the trochanteric fracture area because they brings external fixation pins and not a special external nail which can ask compression or sliding at the trochanteric fracture .

The patent as it is described has the object to eliminate these disadvantages because it brings a special long external screwed nail which inert to the bone by a very small skin incision, with minimal blood loss , which permits the exertion of compression at the trochanteric fracture area or can be left free for sliding as it depends of the fracture type .

With the special connection of changeable angle of the device it is possible to correct easily post-operatively the reduction of the fracture as in cases of varus deformity without need of a second serious open hemorrhagic intervention .

Screwing the nail to the right or to the left it is possible to move the nail inside or outside, post-operatively , without open intervention, which is impossible with the internal osteosynthesis .

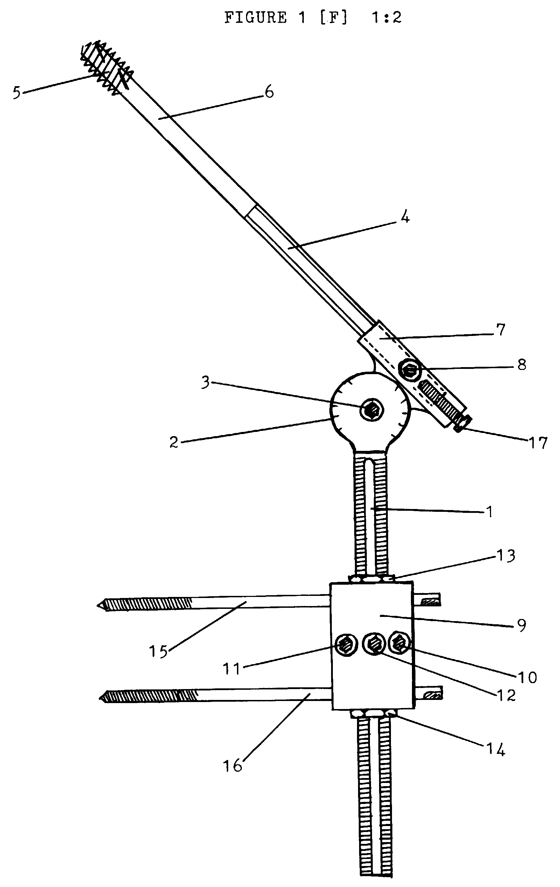

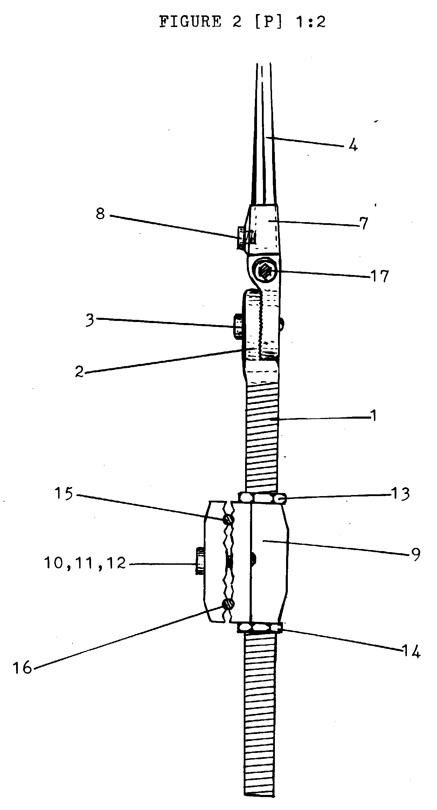

A sort of application of the device is described lower down with references to the drawings of the patent which explain a particular materialization in which the figure (1) presents the front view of the device and the figure (2) the lateral view of the device .

After the close reduction of the fracture under radiological or fluoroscopic control with a small skin incision lower of the greater trochanter and with the aid of a metal guide pin and analogous cannulated reamer the nail (4)of the device is inserted which is cannulated to be inserted on the guide pin and brings screwed end(5) for the stabilization of the head and the neck of the femur. It does not bring thicker part for its stabilization in the cortex of the femoral bone but central part(6) of the same diameter with the rest of the nail so it is possible to be inserted with only one reamer and not with two reamers which are needed in the internal sliding nails .

The nail is connected with its free part outside of the skin with the proximal body of the device (7) and is stabilized on it with the screw (8) or can be left free for sliding or can ask compression on the trochanteric fracture area with the compression screw (17) if it is thought that it is necessary from the type and the form of the fracture .

With the goniometric (changing angle) central connection (2) of the device which is stabilized with the screw (3) it is possible to give different directions at the nail for the anatomical reduction of the fracture .

On the central connection of the device a screwed axis(1) or different type axis is joined for the stabilization of the external fixation pins which permits proximal or distal displacement of the support (9) of the external fixation pins (15 and 16) or exertion of compression parallel to the axis of the device .A such kind of compression can be exercised with the nut (14) after the loosening of the screw (12) and the nut(13).The external fixation pins are stabilized with the screws (10 and 11).As it is mentioned , on the axis of the device, other systems of external fixation pins support can be stabilized , with multiple or simple application of pins, or it is possible to put a support for complementary external fixation pin inserted to the head and the neck of the femoral bone as incases of unstable trochanteric fractures .

## Claims

1. This external fixator with changeable angle for trochanteric fractures , with the possibility of changing the angle of the osteosynthesis in varus or valgus , is characterized by that bears an external long cannulated nail with screwed end for stabilization of the head and the neck of the femoral bone which has the possibility to ask compression to the fracture area or can be left free for sliding .

2. The nail is characterized also by that it is possible to be inserted to the bone by a small skin incision with the help of a guide pin and analogous reamer without bearing central more thicker part for stabilization to the insertion hole on the cortical bone of the trochanteric area of the femoral bone.
